Europäisches Patentamt

European Patent Office

Office européen des brevets

(11) Numéro de publication : **0 423 015 B1**

## (12) FASCICULE DE BREVET EUROPEEN

(45) Date de publication du fascicule du brevet :
**05.05.93 Bulletin 93/18**

(51) Int. Cl.$^5$ : **A61K 7/04**

(21) Numéro de dépôt : **90402798.4**

(22) Date de dépôt : **09.10.90**

(54) Composition effervescente pour les ongles.

(30) Priorité : **13.10.89 FR 8913421**

(43) Date de publication de la demande :
**17.04.91 Bulletin 91/16**

(45) Mention de la délivrance du brevet :
**05.05.93 Bulletin 93/18**

(84) Etats contractants désignés :
**AT BE CH DE FR GB IT LI NL**

(56) Documents cités :
**EP-A- 0 106 173**
**DE-A- 2 300 594**
**DE-A- 3 512 717**
**GB-A- 1 471 679**

(56) Documents cités :
**S.T.N. SERVEUR DE BASES DE DONNEES,
Karlsruhe R.F.A., FICHIER CHEMICAL ABS-
TRACTS, vol. 101, no. 8, résumé no. 59976e; K.
SAKAMOTO: "The development of two new
moisturizing ingredients", & COSMET. TOILE-
TRIES, 99(3), 109-10, 112-14, 116-17**

(73) Titulaire : **L'OREAL**
**14, Rue Royale**
**F-75008 Paris (FR)**

(72) Inventeur : **Ser, Jean-Claude**
**11, rue de Fresnes**
**F-94550 Chevilly-Larue (FR)**
Inventeur : **Martin, Florence**
**1, rue Jules Edouard Voisember**
**F-92130 Issy-Les-Moulineaux (FR)**

(74) Mandataire : **Peuscet, Jacques et al**
**Cabinet Peuscet 68, rue d'Hauteville**
**F-75010 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 423 015 B1

## Description

La présente invention concerne un procédé de préparation d'une solution aqueuse effervescente, ayant une action émolliente sur la cuticule des ongles, destinée à un traitement cosmétique et/ou pharmaceutique ; la présente invention a également pour objet une composition pour la mise en oeuvre de ce procédé et un procédé de traitement cosmétique des ongles à l'aide de la solution aqueuse obtenue.

Les soins de manucure comportent généralement un traitement des ongles pour en éliminer les cuticules, c'est-à-dire la prolongation cornée de l'éponychium recouvrant l'ongle, et donner ainsi un aspect plus soigné aux ongles. Le traitement mécanique traditionnel est déconseillé, car il est considéré comme traumatisant pour les ongles. En remplacement de ce traitement mécanique, on a utilisé des compositions qui se présentent sous forme de solutions, de crèmes ou de gels que l'on met en contact avec la cuticule des ongles et qui ont pour fonction de ramollir et, en partie, détruire celle-ci. Toutefois, les compositions proposées jusqu'à présent sont à base de potasse, de soude ou de borates, qui sont des substances fortement alcalines et, par conséquent, caustiques ; les compositions utilisées sont donc agressives vis-à-vis de l'ongle et de la peau des doigts au voisinage de l'ongle.

Par ailleurs, il est connu d'utiliser des produits effervescents dans le domaine pharmaceutique et cosmétique. On peut citer, par exemple, les cachets effervescents contre les maux de tête ou les sels de bain effervescents. Dans ces produits, l'effervescence est obtenue par réaction entre un bicarbonate, le plus souvent de sodium et un acide RH selon le schéma réactionnel suivant :

$$CO_3HNa + R\text{-}H \rightarrow CO_2\uparrow + Na\text{-}R + H_2O$$

Le fait que les produits soient effervescents rend leur utilisation plus agréable et/ou plus confortable.

La présente invention a pour objet une solution effervescente, qui a une action émolliente sur les cuticules sans être agressive vis-à-vis des ongles et de la peau des doigts au voisinage de l'ongle.

Selon la présente invention, on propose l'utilisation d'une solution aqueuse préparée par introduction sensiblement simultanée dans de l'eau des composés solides anhydres suivants : urée, acide pyrrolidone carboxylique (PCA) et bicarbonate alcalin et/ou d'ammonium.

Lors de leur introduction dans l'eau, l'urée et le bicarbonate alcalin ou d'ammonium se dissolvent. L'acide pyrrolidone carboxylique, qui est peu soluble dans l'eau, réagit progressivement avec le bicarbonate alcalin et/ou d'ammonium en solution pour former le sel alcalin et/ou d'ammonium de l'acide pyrrolidone carboxylique, qui est soluble dans l'eau, avec dégagement de gaz carbonique. Le sel de PCA obtenu est un agent hydratant, dont l'action vient compléter l'action émolliente de l'urée. On obtient ainsi une solution, qui n'est pas agressive vis-à-vis des ongles et de la peau des doigts au voisinage de l'ongle, tout en ayant une action émolliente efficace pour éliminer les cuticules. De plus, par suite du dégagement de gaz carbonique, la solution est effervescente, ce qui ajoute au confort de l'utilisation.

On peut noter que, selon la présente invention, l'acide pyrrolidone carboxylique a une double fonction : produire dans la solution un sel ayant une action hydratante et produire une solution effervescente. Par ailleurs, l'absence totale d'eau dans les composés solides utilisés permet de stocker l'urée en présence des autres produits sans que se posent des problèmes de stabilité.

La présente invention a donc pour objet un procédé de préparation d'une solution aqueuse effervescente ayant une action émolliente sur la cuticule des ongles, destinée à un traitement cosmétique et/ou pharmaceutique, caractérisé par le fait que l'on introduit sensiblement simultanément, dans un milieu aqueux, des composés solides anhydres à raison de 1 g à 15 g pour 100 g de milieu aqueux, dans les proportions suivantes rapportées à 100 parties en poids pour l'ensemble des composés solides anhydres introduits :
- 10 à 25 parties en poids d'urée,
- 50 à 90 parties en poids d'au moins un bicarbonate alcalin et/ou d'ammonium et d'acide pyrrolidone carboxylique (PCA), le rapport pondéral du (ou des) bicarbonate(s) au PCA étant compris entre 1/4 et 4/1 et
- 0 à 40 parties en poids d'au moins un ingrédient complémentaire hydrosoluble choisi dans le groupe formé par les composés, autres que ceux précités, ayant une action émolliente, les composés à action pharmaceutique pour le traitement des ongles et les adjuvants cosmétiquement et/ou pharmaceutiquement acceptables.

Le milieu aqueux utilisé peut contenir un adjuvant cosmétique et/ou pharmaceutique ne réagissant avec aucun des composés solides anhydres et n'ayant pratiquement aucune influence sur leur solubilité. Le milieu aqueux contient, en général plus de 95 % d'eau. De préférence, on utilise de l'eau comme milieu aqueux.

On introduit, de préférence, les composés solides anhydres dans le milieu aqueux sous forme d'un seul mélange ou de plusieurs mélanges partiels préalablement préparés.

On introduit, de préférence, dans le milieux aqueux les composés solides anhydres en adoptant les proportions suivantes, rapportées à 100 parties en poids pour l'ensemble des composés solides anhydres introduits :

- 15 à 20 parties en poids d'urée,
- 70 à 85 parties en poids d'au moins un bicarbonate alcalin et/ou d'ammonium et d'acide pyrrolidone carboxylique (PCA).

Le rapport pondéral du (des) bicarbonate(s) au PCA est, de préférence compris entre 2/3 et 3/2.

Le bicarbonate alcalin est, notamment, un bicarbonate de sodium, de potassium et de lithium.

Parmi les ingrédients complémentaires, on introduit de préférence comme autre(s) composé(s) à activité émolliente au moins un acide aminé, neutre ou basique à raison, en particulier, de 0,5 à 10 % en poids par rapport à l'ensemble des composés solides anhydres. Parmi ces acides aminés on choisit, en particulier, l'arginine, la lysine, l'histidine et la proline.

Parmi les ingrédients complémentaires on introduit comme composé(s) pharmaceutique(s), de préférence, au moins un composé choisi parmi les agents antifongiques, antiseptiques et antimicrobiens hydrosolubles, sous forme cationique, anionique ou non-ionique, le(s)dit(s) composé(s) étant utilisé(s) , selon son (leur) acidité en alcalinité, à raison de 0,1 à 5 % et, plus particulièrement, de 2 à 4 % en poids, par rapport à l'ensemble des composés solides anhydres. On choisit plus particulièrement le chlorure de benzalkonium ou de benzéthonium, le diiséthionate d'hexamédine, le digluconate de chlorhéxidine et les dérivés de pyridinethione sous forme acide, en particulier la 1-hydroxy-2-pyridinethione et, plus particulièrement, les dérivés de pyridinethione vendus sous la marque OMADINE par la Société OLIN CHEMICALS.

On peut introduire également, comme ingrédient complémentaire, un adjuvant cosmétiquement et/ou pharmaceutiquement acceptable utilisé de façon usuelle. On utilise, en particulier, au moins un agent épaississant donnant une légère viscosité à la solution effervescente obtenue, à raison de 0,05 à 3 % en poids par rapport à l'ensemble des composés solides anhydres introduits. Parmi les agents épaississants on peut citer notamment les polymères cationiques et/ou non-ioniques, du type dérivés cellulosiques, et des gommes à gonflement immédiat du type gomme adragante.

Comme expliqué plus en détail ci-après, comme adjuvant cosmétiquement et/ou pharmaceutiquement acceptable, on peut aussi utiliser au moins un liant de compactage à raison de 0 à 30 % en poids par rapport au poids de l'ensemble des composés solides anhydres introduits.

On peut également introduire au moins un colorant et/ou au moins un parfum, notamment adsorbé sur un des solides de la composition.

Pour préparer la solution effervescente selon l'invention on introduit, de préférence, 1 à 10 g de composés solides pour 100 ml de milieu aqueux, mais cette quantité peut être plus élevée, la limite supérieure étant essentiellement fonction de la solubilité de l'ensemble des composés solides anhydres introduits.

Le milieu aqueux est avantageusement à une température comprise entre 15 et 40°C. Le pH de la solution effervescente obtenue est généralement compris entre 7 et 10, plus particulièrement, entre 8,5 et 9,5.

Les composés solides sont introduits sensiblement simultanément dans le milieux aqueux. Le terme "sensiblement simultanément" signifie que l'on introduit les différents composés solides sur une période de temps suffisamment brève pour que les différents composés anhydres solides n'aient pas le temps de réagir entre eux ou de se décomposer de façon notable avant que l'ensemble des composés solides anhydres ne sont introduits dans le milieu aqueux. Cette période de temps ne dépasse pas généralement 5 minutes.

La présente invention a pour second objet une composition de solides anhydres utilisable pour la mise en oeuvre du procédé tel qu'il est défini ci-dessus.

La composition solide pour la mise en oeuvre du procédé est caractérisée par le fait qu'elle comporte, dans un même conditionnement, sous forme d'un seul mélange ou de plusieurs mélanges partiels séparés, pour 100 parties en poids :

- 10 à 25 parties en poids d'urée,
- 50 à 90 parties en poids d'au moins un bicarbonate alcalin ou d'ammonium et d'acide pyrrolidone carboxylique (PCA), le rapport pondéral du (des) bicarbonate(s) au PCA étant compris entre 1/4 et 4/1 et
- O à 40 % en poids d'au moins un ingrédient hydrosoluble complémentaire choisi dans le groupe formé par les composés, autres que ceux précités, ayant une action émolliente, les composés à action pharmaceutique pour le traitement des ongles et les adjuvants cosmétiquement et/ou pharmaceutiquement acceptables.

Cette composition contient de préférence, pour 100 parties en poids :

- 15 à 20 parties en poids d'urée,
- 70 à 85 parties en poids d'au moins un bicarbonate alcalin et/ou d'ammonium et d'acide pyrrolidone carboxylique (PCA).

Le rapport pondéral du (des) bicarbonate(s) au PCA est, de préférence, compris entre 2/3 et 3/2.

Cette composition renferme les ingrédients complémentaires définis précédemment. Comme agent émollient elle peut contenir au moins un acide aminé neutre ou basique présent à raison, en particulier, de 0,5 à 10 % en poids par rapport au poids de l'ensemble des composés solides anhydres. Les composés à action pharmaceutique pour traitement des ongles sont un composé choisi parmi les agents antifongiques, antiseptiques et antimicrobiens hydrosolubles, sous forme cationique, anionique ou non-ionique, le(s)dit(s) composé(s) étant présent(s) à raison de 0,1 à 5 % en poids par rapport à l'ensemble des composés solides anhydres introduits. Les adjuvants cosmétiquement et/ou pharmaceutiquement acceptables peuvent être pris dans le groupe formé par les agents épaississants présents à raison de 0,05 à 3 %, un liant de compactage présent à raison de 0 à 30 %, ces pourcentages étant pris par rapport au poids de l'ensemble des composés solides anhydres introduits.

Dans le cas où le conditionnement contient les composés solides anhydres sous forme de plusieurs mélanges partiels séparés, le(les) bicarbonate(s) d'une part et le PCA d'autre part, sont contenus dans deux mélanges partiels différents. La composition est de préférence composée de deux mélanges partiels différents. Dans ce cas l'urée est avantageusement contenu dans le même mélange partiel B que le PCA et le(les) bicarbonate(s) est (sont) contenu(s) dans l'autre mélange partiel A, les ingrédients complémentaires à caractère acide étant, de préférence, dans le mélange partiel B et ceux ayant un caractère basique dans le mélange partiel A.

Le mélange ou les mélanges de composés solides anhydres de la composition sont sous forme pulvérulente ou sous forme de pastille(s) obtenue(s) par compactage sous pression à partir d'une poudre.

Lorsque la composition comporte plusieurs mélanges pulvérulents elle se présente sous forme d'un conditionnement comportant au moins deux parties séparées. Le conditionnement se présente, par exemple, sous forme d'une boîte contenant au moins deux sachets plastiques, chaque sachet contenant un mélange de composés solides anhydres.

Dans le cas où la composition est sous forme de pastille(s), chaque mélange peut se présenter sous forme de plusieurs pastilles ayant la même composition. Chaque mélange peut également se présenter sous forme d'une pastille unique contenant la quantité dudit mélange nécessaire pour une quantité d'eau déterminée. Par exemple dans le cas d'un mélange unique la pastille peut peser 1 à 15 g pour 100 ml de milieu aqueux. Dans le cas de deux mélanges la composition est sous forme de deux pastilles la première étant, par exemple, préparée à partir du mélange A défini ci-dessus et la seconde à partir du mélange B défini ci-dessus, les pastilles de mélanges A et B pesant au total par exemple 1 à 10 g lorsqu'elles doivent être introduites dans 100 ml de milieu aqueux.

Il n'est pas nécessaire, comme dans le cas des mélanges pulvérulents de conditionner les pastilles correspondant à des mélanges différents dans des parties différentes du conditionnement ; le fait que les mélanges soient sous forme de pastille(s) suffit à séparer les différents mélanges et à éviter que deux composés solides anhydres faisant partie de deux mélanges différents ne risquent de réagir entre eux, au cours d'une conservation en humidité normale.

Les pastilles sont préparées de façon connue par compactage sous pression en présence d'un liant de compactage utilisé à raison de 0 à 30 %. Les liants de compactage utilisés sont des liants généralement connus dans l'industrie pharmaceutique. On peut citer l'amidon, les gommes, en particulier la gomme arabique et la carboxyméthylcellulose. On utilise, en particulier, une cellulose microcristalline, par exemple celle vendu sous la marque AVICEL par FMC Corp. On peut également introduire dans les pastilles des additifs généralement utilisés pour la fabrication de pastilles tels que des charges ou des agents de lissage destinés à donner un aspect lisse et brillant aux pastilles.

Le temps de délitement de la (ou des) pastille(s) est de préférence inférieur à 5 minutes grâce au choix d'une pression de compactage appropriée. Cette pression est variable et dépend de la quantité de liant et du résultat effervescent recherché. Elle est, notamment, comprise entre $2 \times 10^7$ et $2 \times 10^8$ Pascal.

Un troisième objet de la présente invention est un procédé de traitement des ongles à l'aide de la solution aqueuse effervescente obtenue selon le procédé de la présente invention. Selon ce procédé de traitement on met en contact la cuticule des ongles avec la solution aqueuse effervescente pendant un temps suffisant pour obtenir une action émolliente.

Selon ce traitement on trempe les ongles dans la solution aqueuse effervescente juste après l'introduction des composés solides anhydres dans le milieu aqueux et laisse agir ladite solution pendant un temps compris entre 1 et 5 minutes.

Le traitement a un caractère cosmétique car il fait partie des soins de manucure destinés à améliorer l'aspect des ongles.

Il a un caractère pharmaceutique dans la mesure où, lorsque la solution contient un agent pharmaceutique, il permet de traiter les maladies : infections et inflammations du pourtour de l'ongle ou perionyxis ; l'action émolliente de la solution aqueuse effervescente permet au produit pharmaceutique d'agir plus efficacement.

Pour mieux comprendre l'objet de l'invention, on va en décrire maintenant, à titre d'exemples purement illustratifs et non limitatifs, plusieurs modes de mise en oeuvre. Dans les formulations figurant dans ces exemples, les quantités indiquées représentent des proportions relatives.

EXAMPLE 1

On prépare une composition pulvérulente anhydre pour éliminer les cuticules, en pré-traitemet de soins manucures, cette composition étant destinée à être hydratée au moment de l'emploi pour donner une solution effervescente et étant formulée comme suit :

```
- Bicarbonate de sodium ...........        48  g
- Acide pyrrolidone carboxylique ..        32  g
- Urée ............................        17  g
- Dérivé cellulosique cationique
  commercialisé sous la dénomination
  "POLYQUATERNIUM - 10" par la Société
  "UNION CARBIDE" .................         3  g
- Parfum .................... q.s.
- Colorant ................. q.s.
```

Cette composition est conditionnée dans un sachet en plastique soudé de façon à rester à l'abri de l'humidité de l'air.

Au moment de l'emploi, la manucure place 8 g de poudre dans un bol, et elle y ajoute 100 ml d'eau à 30°C. Une solution effervescente est obtenue, dans laquelle la personne désirant les soins manucures plonge simultanément les doigts d'une main, puis les doigts de l'autre main, à chaque fois pendant 2 minutes.

La cuticule ayant subi une action émolliente efficace, il est alors possible à la manucure d'effectuer les soins manucures ultérieurs dans les meilleures conditions. Les doigts sont ensuite rincés.

EXEMPLE 2

On prépare une composition pulvérulente anhydre pour éliminer les cuticules, cette composition, destinée à être hydratée au moment de l'emploi pour donner une solution effervescente, étant formulée comme suit :

```
- Bicarbonate d'ammonium .........        35  g
- Acide pyrrolidone carboxylique ..       50  g
- Urée ............................        10  g
- Polyacrylamide commercialisé sous
  la dénomination "GELAMIDE 250" par
  la Société "AMERICAN CYANAMID" ..        1  g
- L-Arginine ....................         4  g
- Parfum .................... q.s.
- Colorant ................. q.s.
```

La manucure place 3 g de cette poudre dans un bol et elle y ajoute 100 ml d'eau à 30°C. Le traitement des ongles est effectué comme à l'exemple 1 et fournit des résultats analogues.

EXEMPLE 3

On prépare une composition pulvérulente anhydre pour le traitement des périonyxis, cette composition étant destinée à être hydratée au moment de l'emploi pour donner naissance à une solution effervescente et étant formulée comme suit :

5

```
- Bicarbonate de potassium ........      45   g
- Acide pyrrolidone carboxylique ..      30   g
- Urée ..........................         20   g
- Gomme de guar quaternisée, commercialisée


  sous la dénomination "JAGUAR C13" par la
  Société "CELANESE" ..............         1   g
- Chlorure de benzalkonium ........        4   g
- Parfum ................... q.s.
- Colorant ................ q.s.
```

Cette poudre conduit, lorsqu'elle est placée dans l'eau, à une solution effervescente possédant des propriétés traitantes vis-à-vis des périonyxis.

Pour effectuer le traitement d'un périonyxis, on place 5 g de cette poudre dans un bol et on y ajoute 100 ml d'eau à 35°C. Le doigt, présentant une inflammation sur le pourtour de l'ongle est plongé dans la solution effervescente ainsi formée et y est maintenu pendant 4 minutes.

On a recours à ce traitement deux fois par jour pendant 3 jours ce qui conduit à une nette réduction de l'inflammation.

## EXEMPLE 4

On prépare une composition pulvérulente en deux mélanges A et B séparés que l'on introduit simultanément dans l'eau au moment de l'emploi. La composition contient 5O g de mélange A et 50 g de mélange B.

Le mélange A a pour formulation en grammes pour 100 g de mélange.

```
- Bicarbonate de sodium ...........      96      g
- Polyacrylamide vendu sous la
  dénomination "GELAMIDE 25O" par
  la Société "AMERICAN CYANAMID"......    3,5     g
- Parfum .........................       0,5     g
```

Le mélange B a pour formulation en grammes pour 100 g de mélange.

```
- Urée ............................      30      g
- Acide pyrrolidone carboxylique ..     63,9    g
- Polymère commercialisé sous
  la dénomination "POLYMER JR-400"

  par la Société "UNION CARBIDE"...      6      g
- Colorant (Color Index n° 14700)..      0,1    g
```

Les mélanges sont conditionnés dans une même boîte dans deux sachets en matière plastique fermés par soudure contenant au total 10 g de produit, soit 5 g de mélange A et 5 g de mélange B.

La manucure dissout simultanément le contenu des deux sachets dans 100 ml d'eau à 25°C.

Le traitement des ongles est effectué comme à l'exemple 1 et les résultats fournis sont analogues.

EXEMPLE 5

Un comprimé effervescent est préparé à partir du mélange des ingrédients suivants dans les proportions indiquées :

```
    - Bicarbonate de sodium............    36    g
    - Acide pyrrolidone carboxylique....    26    g
    - Cellulose microcristalline vendue
      par la société "FMC CORP." sous la
      dénomination commerciale
      "AVICEL PH105".....................    25    g
    - Urée..............................    12    g
    - Colorant..........................qs
    - Parfum............................qs
    - Arginine..........................     1    g
```

Le mélange est compressé, à l'aide d'une machine de compactage, à $10^8$ Pascal.

Chaque comprimé représente 12 g du mélange. Au moment de l'emploi, il est mis à dissoudre dans 100 ml d'eau à 30°C.

Le traitement des ongles est effectué comme à l'exemple 1 et les résultats fournis sont analogues.

## Revendications

1. Procédé de préparation d'une solution aqueuse effervescente ayant une action émolliente sur la cuticule des ongles, destinée à un traitement cosmétique et/ou pharmaceutique, comprenant sensiblement simultanément l'introduction dans un milieu aqueux, des composés solides anhydres à raison de 1 g à 15 g pour 100 g de milieu aqueux, dans les proportions suivantes rapportées à 100 parties en poids pour l'ensemble des composés solides anhydres introduits :
   - 10 à 25 parties en poids d'urée,
   - 50 à 90 parties en poids d'au moins un bicarbonate alcalin et/ou d'ammonium et d'acide pyrrolidone carboxylique (PCA), le rapport pondéral du (des) bicarbonate(s) au PCA étant compris entre 1/4 et 4/1 et
   - 0 à 40 parties en poids d'au moins un ingrédient complémentaire hydrosoluble choisi dans le groupe formé par les composés, autres que ceux précités, ayant une action émolliente, les composés à action pharmaceutique pour le traitement des ongles et les adjuvants cosmétiquement et/ou pharmaceutiquement acceptables.

2. Procédé selon la revendication 1, caractérisé par le fait qu'on utilise de l'eau comme milieu aqueux.

3. Procédé selon la revendication 1 ou 2, caractérisé par le fait que l'on introduit les composés solides anhydres dans le milieu aqueux sous forme d'un seul mélange ou de plusieurs mélanges partiels préalablement préparés.

4. Procédé selon l'une des revendications 1 à 3, caractérisé par le fait que l'on introduit les composés solides anhydres dans le milieu aqueux en adoptant les proportions suivantes rapportées à 100 parties en poids pour l'ensemble des composés solides anhydres introduits :
   - 15 à 20 parties en poids d'urée,
   - 70 à 85 parties en poids d'au moins un bicarbonate alcalin ou d'ammonium et d'acide pyrrolidone carboxylique (PCA).

5. Procédé selon l'une des revendications 1 à 4, caractérisé par le fait que le rapport pondéral du (des) bicarbonate(s) au PCA est compris entre 2/3 et 3/2.

**6.** Procédé selon l'une des revendications 1 à 5, caractérisé par le fait que, parmi les ingrédients complémentaires, on introduit comme autre(s) composé(s) à activité émolliente, au moins un acide aminé, neutre ou basique, à raison de 0,5 à 10 % en poids par rapport à l'ensemble des composés solides anhydres introduits.

**7.** Procédé selon la revendication 6, caractérisé par le fait qu'on choisit le (les) acide(s) aminé(s) dans le groupe formé par l'arginine, la lysine, l'histidine et la proline.

**8.** Procédé selon l'une des revendications 1 à 7, caractérisé par le fait que, parmi les ingrédients complémentaires, on introduit, comme composé(s) à action pharmaceutique, au moins un composé choisi parmi les agents antifongiques, antiseptiques et antimicrobiens hydrosolubles, sous forme cationique, anionique ou non-ionique, le(s) dit(s) composé(s) étant utilisé(s) à raison de 0,1 à 5 % en poids par rapport à l'ensemble des composés solides anhydres introduits.

**9.** Procédé selon la revendication 8, caractérisé par le fait qu'on choisit le(s) composé(s) à action pharmaceutique dans le groupe formé par le chlorure de benzalkonium ou de benzéthonium, le diiséthionate d'hexamédine, le digluconate de chlorhéxidine, et les dérivés de pyridinethione sous forme acide.

**10.** Procédé selon l'une des revendications 1 à 9, caractérisé par le fait qu'on utilise, comme adjuvant cosmétiquement et/ou pharmaceutiquement acceptable, au moins un agent épaississant donnant une légère viscosité à la solution effervescente obtenue, à raison de 0,05 à 3 % en poids par rapport au poids de l'ensemble des composés solides anhydres introduits et/ou au moins un liant de compactage, à raison de O à 30 % en poids par rapport au poids de l'ensemble des composés solides anhydres introduits et/ou au moins un parfum et/ou au moins un colorant.

**11.** Procédé selon l'une des revendications 1 à 10, caractérisé par le fait que le milieu aqueux est à une température comprise entre 15 et 4O°C.

**12.** Composition solide pour la mise en oeuvre du procédé selon l'une des revendications 1 à 11, comprenant, dans un même conditionnement, sous forme d'un seul mélange ou de plusieurs mélanges partiels séparés, pour 100 parties en poids :
- 10 à 25 parties en poids d'urée,
- 50 à 90 parties en poids d'au moins un bicarbonate alcalin ou d'ammonium et d'acide pyrrolidone carboxylique (PCA), le rapport du (des) bicarbonate(s) au PCA étant compris entre 1/4 et 4/1,
- 0 à 40 parties d'au moins un ingrédient complémentaire hydrosoluble choisi dans le groupe formé par les composés autres que ceux précités, ayant une action émolliente, les composés à action pharmaceutique pour le traitement des ongles et les adjuvants cosmétiquement et/ou pharmaceutiquement acceptables.

**13.** Composition selon la revendication 12, caractérisée par le fait qu'elle comporte, pour 100 parties en poids :
- 15 à 20 parties en poids d'urée,
- 70 à 85 parties en poids d'au moins un bicarbonate alcalin et/ou d'ammonium et d'acide pyrrolidone carboxylique (PCA).

**14.** Composition selon l'une des revendications 12 et 13, caractérisée par le fait que le rapport pondéral du (des) bicarbonate(s) au PCA est compris entre 2/3 et 3/2.

**15.** Composition selon l'une des revendications 12 à 14, caractérisée par le fait que, parmi les ingrédients complémentaires, l'agent émollient est au moins un acide aminé, neutre ou basique, présent à raison de 0,5 à 10 % en poids par rapport au poids de l'ensemble des composés solides anhydres introduits.

**16.** Composition selon l'une des revendications 12 à 15, caractérisée par le fait que, parmi les ingrédients complémentaires, les composés à action pharmaceutique pour le traitement des ongles sont au moins un composé choisi parmi les agents antifongiques, antiseptiques, et antimicrobiens hydrosolubles, sous forme cationique, anionique ou non-ionique, le(s)dit(s) composé(s) étant présent(s) à raison de 0,1 à 5 % en poids par rapport à l'ensemble des composés solides anhydres introduits.

**17.** Composition selon l'une des revendications 12 à 16, caractérisée par le fait que les adjuvants cosmétiquement et/ou pharmaceutiquement acceptables sont pris dans le groupe formé par les agents épaississants présents à raison de 0,05 à 3 % en poids, ces pourcentages étant pris par rapport au poids de l'ensemble des composés solides anhydres introduits, les parfums et les colorants.

18. Composition selon l'une des revendications 12 à 17, dans laquelle les composants solides anhydres sont sous forme de plusieurs mélanges partiels séparés, caractérisée par le fait que le(les) bicarbonate(s) d'une part, et le PCA, d'autre part, sont contenus dans deux mélanges partiels différents.

19. Composition selon la revendication 18, caractérisée par le fait qu'elle est constituée de deux mélanges partiels différents.

20. Composition selon la revendication 19, caractérisée par le fait que l'urée est contenue dans le même mélange partiel B que le PCA et que le (les) bicarbonate(s) est (sont) contenu(s) dans l'autre mélange partiel A, les ingrédients complémentaires à caractère acide étant dans le mélange partiel B et ceux ayant un caractère basique dans le mélange partiel A.

21. Composition selon l'une des revendications 12 à 20, caractérisée par le fait que le (ou les) mélange(s) de composés solides anhydres est (sont) sous forme pulvérulente.

22. Composition selon l'une des revendications 12 à 20, caractérisée par le fait que le mélange (ou les) mélanges de composés solides anhydres est (ou sont) sous forme d'une pastille obtenue par compactage sous pression à partir d'une poudre.

23. Composition selon la revendication 22, caractérisée par le fait qu'elle contient un liant de compactage constituant au plus 30 % en poids par rapport au poids total de la composition.

24. Composition selon la revendication 21, caractérisée par le fait qu'elle se présente sous forme d'un conditionnement comportant au moins deux parties séparées.

25. Composition selon l'une des revendications 22 ou 23, caractérisée par le fait que le temps de délitement de la pastille est inférieur à 5 minutes grâce au choix d'une pression de compactage appropriée.

26. Procédé de traitement cosmétique des ongles à l'aide de la solution aqueuse effervescente obtenue par le procédé de l'une des revendications 1 à 11, comprenant la mise en contact de la cuticule des ongles avec ladite solution pendant un temps suffisant pour obtenir une action émolliente.

27. Procédé selon la revendication 26, caractérisé par le fait que l'on trempe les ongles dans la solution juste aprés l'introduction de la composition selon l'une des revendications 12 à 25, dans le milieu aqueux et que l'on laisse agir ladite solution pendant un temps compris entre 1 et 5 minutes.

**Patentansprüche**

1. Verfahren zur Herstellung einer zur kosmetischen und/oder pharmazeutischen Behandlung bestimmten wäßrigen efferveszierenden Lösung mit weichmachender Wirkung auf die Nagelkutikula, wobei man feste wasserfreie Verbindungen im wesentlichen gleichzeitig in ein wäßriges Milieu in einer Menge von 1 g bis 15 g pro 100 g des wäßrigen Milieus und in den nachfolgenden Mengenanteilen, bezogen auf 100 Gewichtsteile der festen wasserfreien Verbindungen zusammengenommen, gibt:
   - 10 bis 25 Gewichtsteile Harnstoff,
   - 50 bis 90 Gewichtsteile wenigstens eines Alkalimetall- und /oder Ammoniumbicarbonates und Pyrrolidoncarbonsäure (PCA), wobei das Gewichtsverhältnis von Bicarbonat(en) zu PCA zwischen 1/4 und 4/1 liegt und
   - 0 bis 40 Gewichtsteile wenigstens eines ergänzenden wasserlöslichen Bestandteils, der ausgewählt ist unter anderen als den obenerwähnten Verbindungen mit weichmachender Wirkung, Verbindungen mit pharmazeutischer Wirkung zur Behandlung der Nägel und kosmetisch und/oder pharmazeutisch akzeptablen Adjuvantien.

2. Verfahren nach Anspruch 1, dadurch gekennzeichnet, daß man als wäßriges Milieu Wasser verwendet.

3. Verfahren nach Anspruch 1 oder 2, dadurch gekennzeichnet, daß man die festen wasserfreien Verbindungen in Form einer einzigen Mischung oder in Form mehrerer vorher zubereiteter Teilmischungen in das wäßrige Milieu gibt.

4. Verfahren nach einem der Ansprüche 1 bis 3, dadurch gekennzeichnet, daß man die festen wasserfreien Verbindung in den folgenden Mengenanteilen, bezogen auf 100 Gewichtsteile der eingesetzten festen wasserfreien Verbindungen zusammengenommen, in das wäßrige Milieu gibt:
   - 15 bis 20 Gewichtsteile Harnstoff,
   - 70 bis 85 Gewichtsteile wenigstens eines Alkalimetall- oder Ammoniumbicarbonates und Pyrrolidoncarbonsäure (PCA).

5. Verfahren nach einem der Ansprüche 1 bis 4, dadurch gekennzeichnet, daß das Gewichtsverhältnis von Bicarbonat(en) zu PCA zwischen 2/3 und 3/2 liegt.

6. Verfahren nach einem der Ansprüche 1 bis 5, dadurch gekennzeichnet, daß man als zu den ergänzenden Bestandteilen zählende(n) andere(n) Verbindung(en) mit weichmachender Wirkung wenigstens eine neutrale oder basische Aminosäure in einer Menge von 0,5 bis 10 Gew.-%, bezogen auf die eingesetzten festen wasserfreien Verbindungen zusammengenommen, zugibt.

7. Verfahren nach Anspruch 6, dadurch gekennzeichnet, daß die Aminosäure(n) ausgewählt ist (sind) unter Arginin, Lysin, Histidin und Prolin.

8. Verfahren nach einem der Ansprüche 1 bis 7, dadurch gekennzeichnet, daß man als zu den ergänzenden Bestandteilen zählende Verbindung(en) mit pharmazeutischer Wirkung wenigstens eine Verbindung zugibt, die ausgewählt ist unter wasserlöslichen antifungischen, antiseptischen und antimikrobiellen Mitteln in kationischer, anionischer oder nicht-ionischer Form, wobei man die Verbindung(en) in einer Menge von 0,1 bis 5 Gew.-%, bezogen auf die eingesetzten festen wasserfreien Verbindungen zusammengenommen, verwendet.

9. Verfahren nach Anspruch 8, dadurch gekennzeichnet, daß man die Verbindung(en) mit pharmazeutischer Wirkung auswählt unter Benzalkonium- oder Benzethoniumchlorid, Hexamedindiisethionat, Chlorhexidindigluconat und Pyrridinthion-derivaten in Säureform.

10. Verfahren nach einem der Ansprüche 1 bis 9, dadurch gekennzeichnet, daß man als kosmetisch und/oder pharmazeutisch akzeptables Adjuvans wenigstens ein Verdickungsmittel, das die erhaltene efferveszierende Lösung leicht viskos macht, in einer Menge von 0,05 bis 3 Gew.-%, bezogen auf das Gewicht der eingesetzten festen wasserfreien Verbindungen zusammengenommen, und/oder wenigstens ein Kompaktierbindemittel in einer Menge von 0 bis 30 Gew.-%, bezogen auf das Gewicht der eingesetzten wasserfreien Verbindungen zusammengenommen, und/oder wenigstens ein Parfüm und/oder wenigstens einen Farbstoff verwendet.

11. Verfahren nach einem der Ansprüche 1 bis 10, dadurch gekennzeichnet, daß das wäßrige Milieu eine Temperatur zwischen 15 und 40°C hat.

12. Festes Mittel zur Durchführung des Verfahrens nach einem der Ansprüche 1 bis 11, umfassend in der gleichen Aufmachung in Form einer einzigen Mischung oder in Form mehrerer getrennter Teilmischungen pro 100 Gewichtsteile:
   - 10 bis 25 Gewichtsteile Harnstoff
   - 50 bis 90 Gewichtsteile wenigstens eines Alkalimetall- oder Ammoniumbicarbonates und Pyrrolidoncarbonsäure (PCA), wobei das Gewichtsverhältnis von Bicarbonat(en) zu PCA zwischen 1/4 und 4/1 liegt,
   - 0 bis 40 Gewichtsteile wenigstens eines ergänzenden wasserlöslichen Bestandteils, der ausgewählt ist unter anderen als den oben erwähnten Verbindungen mit weichmachender Wirkung, Verbindungen mit pharmazeutischer Wirkung zur Behandlung von Nägeln und kosmetisch und/oder pharmazeutisch verträglichen Adjuvantien.

13. Mittel nach Anspruch 12, dadurch gekennzeichnet, daß es pro 100 Gewichtsteile umfaßt:
   - 15 bis 20 Gewichtsteile Harnstoff,
   - 70 bis 85 Gewichtsteile wenigstens eines Alkalimetall- und /oder Ammoniumbicarbonats und Pyrrolidoncarbonsäure (PCA).

**14.** Mittel nach einem der Ansprüche 12 und 13, dadurch gekennzeichnet, daß das Gewichtsverhältnis von Bicarbonat(en) zu PCA zwischen 2/3 und 3/2 liegt.

**15.** Mittel nach einem der Ansprüche 12 bis 14, dadurch gekennzeichnet, daß es als zu den ergänzenden Bestandteilen zählendes weichmachendes Mittel wenigstens eine neutrale oder basische Aminosäure enthält, die in einer Menge von 0,5 bis 10 Gew.-%, bezogen auf das Gewicht der eingesetzten festen wasserfreien Verbindungen zusammengenommen, vorhanden ist.

**16.** Mittel nach einem der Ansprüche 12 bis 15, dadurch gekennzeichnet, daß es als zu den ergänzenden Bestandteilen zählende Verbindungen mit pharmazeutischer Wirkung zur Behandlung der Nägel wenigstens eine Verbindung enthält, die ausgewählt ist unter wasserlöslichen antifungischen, antiseptischen und antimikrobiellen Mitteln in kationischer, anionischer oder nicht-ionischer Form, wobei die Verbindung(en) in einer Menge von 0,1 bis 5 Gew.-%, bezogen auf die eingesetzten wasserfreien Verbindungen zusammengenommen, vorhanden ist (sind).

**17.** Mittel nach einem der Ansprüche 12 bis 16, dadurch gekennzeichnet, daß die kosmetisch und/oder pharmazeutisch akzeptablen Adjuvantien ausgewählt sind unter Verdickungsmitteln, die in einer Menge von 0,05 bis 3 Gew.-% vorhanden sind, wobei sich diese Prozentangabe auf das Gewicht der eingesetzten festen wasserfreien Verbindungen zusammen-genommen bezieht, Parfüms und Farbstoffen.

**18.** Mittel nach einem der Ansprüche 12 bis 17, wobei die festen wasserfreien Verbindungen in Form mehrerer getrennter Teilmischungen vorliegen, dadurch gekennzeichnet, daß das Bicarbonat (die Bicarbonate) einerseits und PCA andererseits in unterschiedlichen Teilmischungen enthalten sind.

**19.** Mittel nach Anspruch 18, dadurch gekennzeichnet, daß es aus zwei unterschiedlichen Teilmischungen besteht.

**20.** Mittel nach Anspruch 19, dadurch gekennzeichnet, daß der Harnstoff in der gleichen Teilmischung B wie das PCA enthalten ist und daß das Bicarbonat (die Bicarbonate) in der anderen Teilmischung A enthalten ist (sind), wobei die ergänzenden Bestandteile mit saurem Charakter in der Teilmischung B und diejenigen mit basischem Charakter in der Teilmischung A enthalten sind.

**21.** Mittel nach einem der Ansprüche 12 bis 20, dadurch gekennzeichnet, daß die Mischung(en) der festen wasserfreien Verbindungen in Pulverform vorliegt (vorliegen).

**22.** Mittel nach einem der Ansprüche 12 bis 20, dadurch gekennzeichnet, daß die Mischung(en) der festen wasserfreien Verbindungen in Form einer Pastille vorliegt (vorliegen), welche ausgehend von einem Pulver durch Kompaktieren unter Druck erhalten worden ist.

**23.** Mittel nach Anspruch 22, dadurch gekennzeichnet, daß es ein Kompaktierbindemittel enthält, das höchstens 30 Gew-%, bezogen auf das Gesamtgewicht des Mittels, ausmacht.

**24.** Mittel nach Anspruch 21, dadurch gekennzeichnet, daß es in Form einer Aufmachung vorliegt, die wenigstens zwei getrennte Teile umfaßt.

**25.** Mittel nach einem der Ansprüche 22 oder 23, dadurch gekennzeichnet, daß die Zerfallszeit der Pastille durch die Wahl eines geeigneten Kompaktierdrucks weniger als 5 Minuten beträgt.

**26.** Verfahren zur kosmetischen Behandlung der Nägel mit Hilfe der wäßrigen efferveszierenden Lösung, die nach dem Verfahren nach einem der Ansprüche 1 bis 11 erhalten wurde, wobei man die Nagelkutikula mit der Lösung während einer Zeit in Kontakt bringt, die ausreicht, um eine weichmachende Wirkung herbeizuführen.

**27.** Verfahren nach Anspruch 26, dadurch gekennzeichnet, daß man die Nägel in die Lösung unmittelbar nach Einbringen des Mittels nach einem der Ansprüche 12 bis 25 in das wäßrige Milieu eintaucht und daß man die Lösung während einer Zeit zwischen 1 und 5 Minuten einwirken läßt.

**Claims**

1. Process for preparing an effervescent aqueous solution having an emollient action on the cuticle of the nails, intended for a cosmetic and/or pharmaceutical treatment, comprising substantially simultaneously the introduction of anhydrous solid compounds into an aqueous medium on the basis of 1 g to 15 g per 100 g of aqueous medium, in the following proportions referred to 100 parts by weight for the collective anhydrous solid compounds introduced:
   - 10 to 25 parts by weight of urea,
   - 50 to 90 parts by weight of at least one alkali metal and/or ammonium bicarbonate and pyrrolidone-carboxylic acid (PCA), the weight ratio of the bicarbonate(s) to PCA being between 1:4 and 4:1, and
   - 0 to 40 parts by weight of at least one additional water-soluble ingredient selected from the group composed of compounds, other than those mentioned above, having an emollient action, compounds having a pharmaceutical action for the treatment of the nails and cosmetically and/or pharmaceutically acceptable adjuvants.

2. Process according to Claim 1, characterised in that water is used as an aqueous medium.

3. Process according to Claim 1 or 2, characterised in that the anhydrous solid compounds are introduced into the aqueous medium in the form of a single mixture or of several partial mixtures prepared beforehand.

4. Process according to one of Claims 1 to 3, characterised in that the anhydrous solid compounds are introduced into the aqueous medium adopting the following proportions referred to 100 parts by weight for the collective anhydrous solid compounds introduced:
   - 15 to 20 parts by weight of urea, and
   - 70 to 85 parts by weight of at least one alkali metal or ammonium bicarbonate and pyrrolidone-carboxylic acid (PCA).

5. Process according to one of Claims 1 to 4, characterised in that the weight ratio of the bicarbonate(s) to PCA is between 2:3 and 3:2.

6. Process according to one of Claims 1 to 5, characterised in that, among additional ingredients, as (an)other compound(s) having emollient activity, at least one neutral or basic amino acid is introduced, on the basis of 0.5 to 10 % by weight relative to the collective anhydrous solid compounds introduced.

7. Process according to Claim 6, characterised in that the amino acid(s) is/are selected from the group composed of arginine, lysine, histidine and proline.

8. Process according to one of Claims 1 to 7, characterised in that, among additional ingredients, as (a) compound(s) having a pharmaceutical action, at least one compound selected from water-soluble antifungal, antiseptic and antimicrobial agents, in cationic, anionic or nonionic form, is introduced, the said compound(s) being used on the basis of 0.1 to 5 % by weight relative to the collective anhydrous solid compounds introduced.

9. Process according to Claim 8, characterised in that the compound(s) having a pharmaceutical action are selected from the group composed of benzalkonium or benzethonium chloride, hexamidine diisethionate, chlorhexidine digluconate and pyridinethione derivatives in acid form.

10. Process according to one of Claims 1 to 9, characterised in that, as a cosmetically and/or pharmaceutically acceptable adjuvant, at least one thickening agent giving a slight viscosity to the effervescent solution obtained is used on the basis of 0.05 to 3 % by weight relative to the weight of the collective anhydrous solid compounds introduced, and/or at least one compaction binder is used on the basis of 0 to 30 % by weight relative to the weight of the collective anhydrous solid compounds introduced, and/or at least one fragrance and/or at least one colouring is/are used.

11. Process according to one of Claims 1 to 10, characterised in that the aqueous medium is at a temperature of between 15 and 40°C.

12. Solid composition for carrying out the process according to one of Claims 1 to 11, comprising in the same pack, in the form of a single mixture or of several separate partial mixtures, per 100 parts by weight:
- 10 to 25 parts by weight of urea,
- 50 to 90 parts by weight of at least one alkali metal or ammonium bicarbonate and pyrrolidonecarboxylic acid (PCA), the ratio of the bicarbonate(s) to PCA being between 1:4 and 4:1, and
- 0 to 40 parts of at least one additional water-soluble ingredient selected from the group composed of compounds, other than those mentioned above, having an emollient action, compounds having a pharmaceutical action for the treatment of the nails and cosmetically and/or pharmaceutically acceptable adjuvants.

13. Composition according to Claim 12, characterised in that it contains, per 100 parts by weight:
- 15 to 20 parts by weight of urea, and
- 70 to 85 parts by weight of at least one alkali metal and/or ammonium bicarbonate and pyrrolidonecarboxylic acid (PCA).

14. Composition according to one of Claims 12 and 13, characterised in that the weight ratio of the bicarbonate(s) to PCA is between 2:3 and 3:2.

15. Composition according to one of Claims 12 to 14, characterised in that, among additional ingredients, the emollient agent is at least one neutral or basic amino acid, present on the basis of 0.5 to 10 % by weight relative to the weight of the collective anhydrous solid compounds introduced.

16. Composition according to one of Claims 12 to 15, characterised in that, among additional ingredients, the compounds having a pharmaceutical action for treatment of the nails are at least one compound selected from water-soluble antifungal, antiseptic and antimicrobial agents, in cationic, anionic or nonionic form, the said compound(s) being present on the basis of 0.1 to 5 % by weight relative to the collective anhydrous solid compounds introduced.

17. Composition according to one of Claims 12 to 16, characterised in that the cosmetically and/or pharmaceutically acceptable adjuvants are selected from the group composed of thickening agents present on the basis of 0.05 to 3 % by weight, these percentages being taken relative to the weight of the collective anhydrous solid compounds introduced, fragrances and colourings.

18. Composition according to one of Claims 12 to 17, in which the anhydrous solid components are in the form of several separate partial mixtures, characterised in that the bicarbonate(s) on the one hand and the PCA on the other hand are contained in two different partial mixtures.

19. Composition according to Claim 18, characterised in that it consists of two different partial mixtures.

20. Composition according to Claim 19, characterised in that the urea is contained in the same partial mixture B as the PCA, and in that the bicarbonate(s) is/are contained in the other partial mixture A, the additional ingredients of an acidic nature being in the partial mixture B and those having a basic nature in the partial mixture A.

21. Composition according to one of Claims 12 to 20, characterised in that the mixture(s) of anhydrous solid compounds is/are in pulverulent form.

22. Composition according to one of Claims 12 to 20, characterised in that the mixture or mixtures of anhydrous solid compounds is/are in the form of a tablet obtained from a powder by compaction under pressure.

23. Composition according to Claim 22, characterised in that it contains a compaction binder constituting at most 30 % by weight relative to the total weight of the composition.

24. Composition according to Claim 21, characterised in that it is presented in the form of a pack containing at least two separate parts.

25. Composition according to one of Claims 22 and 23, characterised in that the disintegration time of the tablet is less than 5 minutes as a result of the choice of a suitable compaction pressure.

26. Process for cosmetic treatment of the nails using the effervescent aqueous solution obtained by the process of one of Claims 1 to 11, comprising the cuticle of the nails being brought into contact with the said solution for a sufficient time to obtain an emollient action.

27. Process according to Claim 26, characterised in that the nails are dipped into the solution immediately after the introduction of the composition according to one of Claims 12 to 25 into the aqueous medium, and in that the said solution is left to act for a time of between 1 and 5 minutes.